# EUROPEAN PATENT APPLICATION

(11) **EP 3 722 809 A1**
(43) Date of publication of application: **14.10.2020**
(21) Application number: 18886235.3
(22) Date of filing: 07.12.2018
(51) Int. Cl.: G01N 33/574, G01N 33/53

(54) **METHOD FOR PREDICTING GLIOMA**

(30) Priority: 08.12.2017 JP 2017235811
(71) Applicant: Muragaki, Yoshihiro, Tokyo 162-8666 (JP)
(72) Inventor: MURAGAKI, Yoshihiro, Tokyo 162-8666 (JP); OKAMOTO, Saori, Tokyo 162-8666 (JP); MARUYAMA, Takashi, Tokyo 162-8666 (JP)
(74) Representative: Cabinet Beau de Loménie
(86) International application number: PCT/JP2018/045169
(87) International publication number: WO 2019/112056

(57) **Abstract**

The present invention provides a method for predicting the onset of glioma in a subject. Specifically, the present invention provides a method for predicting the onset of glioma in a subject, comprising the steps of: (1) detecting vimentin in a blood sample derived from the subject; and (2) in a case where vimentin is detected, predicting that there is a high possibility that the subject has contracted glioma.

## Description

### Technical Field

The present invention relates to a method for predicting the onset of glioma in a subject.

### Background Art

Glioma is one of the malignant tumors that occur in the brain and accounts for about 25% of all the intracranial tumors.

There are various types of glioma, and the Classification made by the World Health Organization (WHO) is used worldwide (Non Patent Literature 1). The WHO Classification is based on malignancy and these types of glioma are classified into grade 1 (having the lowest malignancy) to grade 4 (having the highest malignancy). The treatment strategy for each type of glioma is also determined based on these grades.

Since some of glioma have high malignancy and poor prognosis (for example, glioblastoma, which is classified into the grade 4, has a five-year survival rate of 10% or less), it is important to correctly judge that the tumor is glioma and start the treatment suitable for glioma, for the prognosis of patients.

The definitive diagnosis of glioma involves diagnostic imaging such as CT and MRI and histopathologic examination in which the pathologist observes, with microscope, brain tissues sampled from a subject who is suspected of having glioma from the diagnostic imaging.

However, since the histopathologic examination requires complicated operations of immunohistochemical staining, it takes time to obtain the result. In addition, the brain tissue sampling also has high invasiveness and high risk to a subject, there is a case where the brain tissue sampling cannot be conducted.

In diagnostic imaging, it is quite often difficult to distinguish glioma from metastatic brain tumors and malignant lymphoma, which are diseases different from glioma. In addition, since the diagnostic imaging uses contrast agents and takes a long test time, it is difficult to frequently conduct the diagnostic imaging on children and subjects having severe systemic conditions.

To solve these problems, the search for tumor markers contained in bloods, which are easily sampled, has been made and several glioma markers have been reported (Non Patent Literature 2).

As to vimentin, which is employed in the present invention, it has been reported that vimentin can be used as a marker for detecting colonic neoplasms such as colon cancer (Patent Literature 1) and that vimentin can be used as a marker for diagnosing metastatic brain tumors (Patent Literature 2). Note that glioma is not classified into metastatic brain tumors. Furthermore, vimentin has been reported to be expressed in immature neuroglial cells in the central nerves and the expression enhancement of vimentin correlates with the malignancy of glioma, based on the immunohistochemical studies (Non Patent Literature 3). However, it has not been known that vimentin is present out of cells of blood and the like.

### Citation List

### Patent Literatures

Patent Literature 1: International Publication No. 2005/017207
Patent Literature 2: Korean Patent Application Publication No. 10-2014-0108910

### Non Patent Literatures

Non Patent Literature 1: Acta Neuropathol (2007) 114:97-109
Non Patent Literature 2: Neuro-Oncology 17(3), 343-360, 2015
Non Patent Literature 3: NOU SINKEI (Clanial Nerve) (1987) 39: 579-585

### Summary of Invention

### Problems to be solved by the invention

The glioma markers in blood reported so far have low sensitivity and specificity, and accordingly have not been clinically applied.

### Means for solution of the problems

As a result of earnestly studying about means that has a low invasiveness and risk to a subject and is capable of predicting the onset of glioma simply, promptly, and accurately, the present inventors found that the presence of vimentin in the blood of a subject is involved in the onset of glioblastoma. The present invention has been made based on this finding.

Specifically, the present invention relates to the following [1] to [16]:
[1] A method for predicting the onset of glioma in a subject, comprising the steps of:
   (1) detecting vimentin in a blood sample derived from the subject; and
   (2) in a case where vimentin is detected, predicting that there is a high possibility that the subject has contracted glioma.
[2] The method according to [1], wherein
   the glioma is glioma of grade 2 to 4.
[3] The method according to [2], wherein
   the glioma is glioma of grade 4.
[4] The method according to [3], wherein
   the glioma is glioblastoma.
[5] The method according to any one of [1] to [4], wherein
   the subject is a subject who is suspected of having glioma.
[6] The method according to any one of [1] to [4], wherein
   the subject is a subject who is not suspected of having glioma.
[7] The method according to any one of [1] to [6], wherein
   the blood sample is a blood serum sample.
[8] The method according to any one of [1] to [7], wherein
   vimentin is detected by ELISA.
[9] The method according to [8], wherein
   the ELISA is sandwich ELISA.
[10] A blood marker for detecting glioma, comprising vimentin.
[11] The blood marker according to [10], wherein
   the glioma is glioma of grade 2 to 4.
[12] The blood marker according to [11], wherein
   the glioma is glioma of grade 4.
[13] The blood marker according to [12], wherein
   the glioma is glioblastoma.
[14] A kit for predicting the onset of glioma, comprising:
   a reagent for detecting vimentin in a blood sample; and
   an instruction stating that in a case where vimentin is detected, it is predicted that there is a high possibility that a subject has contracted glioma.
[15] The kit according to [14], wherein
   the reagent for detecting vimentin contains a vimentin antibody.
[16] The kit according to [15], wherein
   the reagent for detecting vimentin further contains a reagent used in ELISA.

### Advantageous Effects of Invention

As indicated by Example described below, the present invention is capable of simply, promptly, and accurately predicting the onset of glioma by using a blood, which can be obtained by means that has low invasiveness and risk to a subject. Therefore, using the present invention makes it possible to conduct prompt and accurate diagnosis of glioma on a subject who is suspected of having glioma from diagnostic imaging. In addition, the present invention also makes it possible to conduct an examination for glioma during a normal health examination.

Furthermore, the prediction result obtained by the present invention may be utilized for determination on the effect of the treatment of glioma and determination on the relapse after the treatment.

### Brief Description of Drawings

Fig. 1 is a diagram illustrating the vimentin concentrations in blood serum samples derived from healthy individuals (group 0), patients of grade 2 glioma (group 2), patients of grade 3 glioma (group 3), and patients of grade 4 glioma (group 4).
Fig. 2 is a diagram illustrating the vimentin concentrations in the blood serum samples derived from the healthy individuals (group 0) and the patients of glioma (group 2 to 4).
Fig. 3 is a ROC curve on the blood serum vimentin concentration, created using data of the healthy individuals (group 0) and the patients of glioma (group 2 to 4).

### Description of Embodiments

The present invention will be described in further detail.

A method for predicting the onset of glioma in a subject according to the present invention, comprises the steps of:
(1) detecting vimentin in a blood sample derived from the subject; and
(2) in a case where vimentin is detected, predicting that there is a high possibility that the subject has contracted glioma.

### [Step (1)]

Glioma is one of the malignant tumors that occur in the brain. Brain tumors are broadly divided into primary brain tumors and metastatic brain tumors, and glioma is classified into the primary brain tumor.

According to the WHO Classification based on malignancy, glioma is classified into grade 1 (having the lowest malignancy) to grade 4 (having the highest malignancy).

The grade 1 glioma includes pilocytic astrocytoma and subependymal giant cell astrocytoma.

The grade 2 glioma includes diffuse astrocytoma, oligodendroglioma, and mixed oligodendroglioma-astrocytoma.

The grade 3 glioma includes anaplastic astrocytoma, anaplastic oligodendroglioma, and anaplastic mixed oligodendroglioma-astrocytoma.

The grade 4 glioma includes glioblastoma and gliosarcoma.

The present invention is applicable to glioma of any of these grades, but is applicable preferably to grade 2 to 4 gliomas, and particularly preferably to grade 4 glioma.

Glioblastoma is a glioma that occurs from the neuroglial cells, and is a glioma having a high malignancy that is classified into grade 4 in the WHO Classification. It is known that glioblastoma is likely to occur in the cerebral hemispheres of 45- to 65-year-old males, and as symptoms causes headache, convolution, character change, dementia, paralysis, and the like, and that the progress of the symptoms is fast because of its high malignancy.

The subject includes any animal that could contract glioma, but is preferably a mammal, and particularly preferably a human.

The subject may be one who is suspected of having glioma by publicly-known means for testing glioma (for example, CT, MRI, or the like) or may be one who is not suspected of having glioma (for example, one who has a normal health examination).

In addition, the subject may be of any sex and any age.

The blood sample derived from the subject may be prepared by a method used in the art in general with no particular limitation.

As the blood sample, blood sampled from the subject may be used as it is, but it is preferable to use a sample obtained by processing blood sampled from the subject (for example, blood serum, plasma, or the like). From the viewpoint that it is possible to accurately and promptly measure vimentin, it is preferable to use a blood serum sample.

The blood serum sample may be prepared by a method used in the art in general with no particular limitation. For example, the blood serum sample may be prepared by sampling blood (for example, venous blood) from the subject, allowing the blood standing at room temperature (for example, 30 minutes), and centrifuging this (for example, for 10 minutes at 3500 rpm). The blood serum sample thus prepared may be cryopreserved (for example, -80 ° C) until used for the method of the present invention.

Vimentin is a publicly-known cytoskeletal protein (type III intermediate filament) that is present mainly in the cytoplasms of mesenchymal cells. Although vimentin is currently widely used in immunohistochemical staining, it has not been known that vimentin is present out of cells of blood and the like.

Vimentin in the blood sample may be detected by using a vimentin detection method and a quantification method that are used in the art in general and are applicable to bloods with no particular limitation. Specific examples of the methods include the ELISA (Enzyme-Linked ImmunoSorbent Assay) using a vimentin-specific antibody, which has a high specificity to vimentin and is excellent in quantitativity.

Hence, Step (1) may be executed using vimentin detecting means that is publicly known in the art and is applicable to bloods, for example, a vimentin detection kit including a vimentin antibody, preferably , the ELISA kit for vimentin.

The ELISA favorable for Step (1) includes the sandwich method and the direct-adsorption method. The sandwich ELISA is preferable from the viewpoint that the sandwich ELISA has a high specificity to vimentin.

For example , the sandwich ELISA may be conducted by the following steps:
1. Cause a solid phase to adsorb a vimentin antibody (capture antibody).
2. Block the solid phase.
3. Add the blood sample derive from the subject to the solid phase to form a conjugate of the capture antibody and vimentin.
4. Add an antibody (primary antibody) to biotinylated vimentin to the solid phase to form a conjugate of the capture antibody, vimentin, and the primary antibody.
5. Add an avidinylated enzyme to form a conjugate of the capture antibody, vimentin, the primary antibody, and the enzyme.
6. Add an enzyme substrate to detect an enzyme reaction product.

The primary antibody used in the sandwich ELISA is an antibody to vimentin, which recognizes an epitope different from an epitope recognized by the capture antibody.

Kit for measuring vimentin using the sandwich ELISA are commercially available (for example, trade name: Vimentin ELISA Kit (Uscn Life Science Inc., Wuhan, China)), and any of these commercial products may be used for Step (1) with no particular limitation.

Vimentin in the blood sample may be detected by a method other than the ELISA. Specific examples of such method include a method utilizing micro-flow channels, the western blot method, the dot blot method, the Ouchterloney method, the immunoprecipitation method, and the like.

### [Step (2)]

In Step (2), a possibility that the subject has contracted glioma is predicted based on the presence or absence of vimentin in the blood sample measured in Step (1). Specifically, in a case where vimentin is detected, it is predicted that there is a high possibility that the subject has contracted glioma.

The prediction result obtained according to the present invention may be utilized as described below.

In the case where the present invention is executed on a subject who is suspected of having glioma from diagnostic imaging such as CT or MRI, by combining the prediction result obtained by the present invention with other diagnostic criteria (result of histopathologic examination and the like) for glioma, it is possible to conduct more prompt and more accurate definitive diagnosis. In addition, in the case where brain tissue sampling is difficult due to the health status or the like of the subject, it is possible to more effectively utilize the prediction result by the present invention.

In the case where the present invention is executed on someone who is not suspected of having glioma (for example, someone who has a normal health examination), it is possible to determine whether or not a secondary examination (close examination) is needed for glioma, by using the prediction result obtained according to the present invention. Hence, the test for glioma, which has conventionally not been conducted, may be made to be an examination item in a normal health examination by using the present invention that uses a blood sample, which imposes low invasiveness and risk to a subject, and can be executed with simple means.

Moreover, in the case where a blood sample derived from a subject who has been treated for glioma is used, it is possible to utilize the prediction result obtained according to the present invention for determination on the effect of the treatment and determination on the relapse after the treatment.

The above-described prediction method can be grasped as a blood marker for detecting glioma, comprising vimentin. This blood marker can be used for predicting or detecting the onset of glioma in a subject.

Furthermore, the present invention relates to a kit for conducting the above-described method for predicting glioma. This kit comprises: a reagent for detecting vimentin in a blood sample; and an instruction stating that in a case where vimentin is detected, it is predicted that there is a high possibility that a subject has contracted glioma.

As the reagent for detecting vimentin, any reagent that is used in the art in general for detecting and quantifying vimentin and is applicable to bloods may be used with no particular limitation. Specific example of such reagent includes a vimentin-specific antibody.

The kit of the present invention preferably uses the above-described ELISA, particularly the sandwich ELISA. The kit in this case may comprise a reagent used in ELISA (for example, a solid-phase blocking agent used in the sandwich ELISA, a vimentin antibody (capture antibody), a primary antibody, an enzyme, an enzyme substrate, and the like).

Next, the effects of the present invention will be described in detail by way of Example ; however, the present invention is not limited to Example.

### Example

### [Example 1]

The vimentin concentrations in blood samples derived from glioma patients of grade 2 to 4 according to the WHO Classification and healthy individuals were measured and compared.

### (1) Subjects

The subjects were classified into the following groups.

### [Group 0]

Healthy volunteers: 9 volunteers

### [Group 2]

Glioma of grade 2: 6 patients
where:
Diffuse astrocytoma: 1 patient
Oligodendroglioma: 5 patients

### [group 3]

Glioma of grade 3: 8 patients where:
Anaplastic oligodendroglioma: 8 patients

### [group 4]

Glioma of grade 4: 15 patients
where:
Glioblastoma: 15 patients

The subjects of group 2 to 4 were subjected to the craniotomy and tumor resection or biopsy at the neurosurgery department of the Tokyo Women's Medical University Hospital between May, 2013 and June, 2014 and were definitively diagnosed with the respective diseases from the pathological examinations of sampled brain tissues.

### (2) Preparation of blood samples

The venous blood was sampled from each subject and was left to stand for 30 minutes at room temperature, followed by centrifugation (for 10 minutes at 3500 rpm) to prepare a blood serum sample. The prepared blood serum sample was cryopreserved (-80°C) until vimentin was measured.

### (3) Measurement of vimentin

The cryopreserved blood serum sample was thawed at 4°C. Subsequently, the sandwich ELISA was conducted using a commercially-available vimentin measuring kit (trade name: Vimentin ELISA Kit (Uscn Life Science Inc., Wuhan, China)) according to the protocol attached to the kit to measure the vimentin concentration in the blood serum sample.

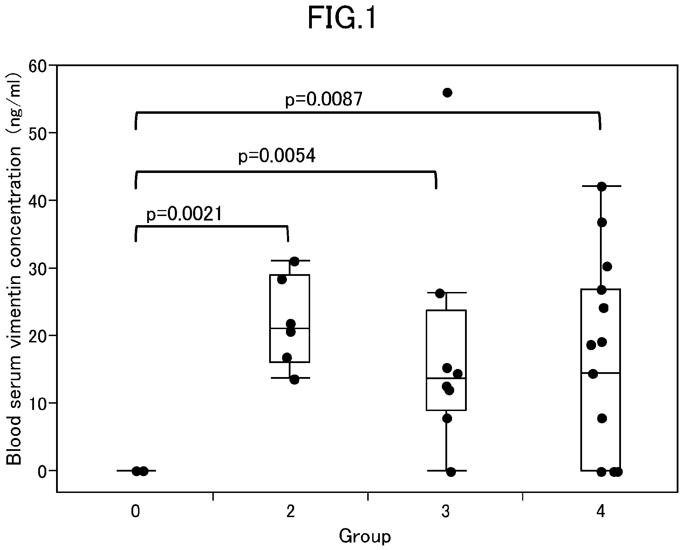

The measurement result is shown in Fig. 1. The vimentin concentration of group 0 was less than the detection limit concentration (7.8 ng/ml) and was thus deemed as 0 ng/ml.

The mean value of the blood serum vimentin concentrations of each group was shown below.

| | Vimentin concentration in blood serum sample (ng/ml) |
|---|---|
| Group 0 (Healthy individuals) | 0 |
| Group 2 (Glioma of grade 2) | 22.0 |
| Group 3 (Glioma of grade 3) | 18.1 |
| Group 4(Glioma of grade 4) | 14.7 |
| Average of Groups 2 to 4 | 17.2 |

The blood serum vimentin concentration among groups was statistically evaluated using the Student-t test. The result is shown in the following table.

**Comparison for each pair in the Student-t test**

| Level | -Level | Difference | Standard error of the difference | Lower confidence limit | Upper confidence limit | p-value |
|---|---|---|---|---|---|---|
| 2 | 0 | 22.02650 | 6.598320 | 8.61710 | 35.43590 | 0.0021 |
| 3 | 0 | 18.08563 | 6.083350 | 5.72277 | 30.44848 | 0.0054 |
| 4 | 0 | 14.70213 | 5.278656 | 3.97461 | 25.42965 | 0.0087 |
| 2 | 4 | 7.32437 | 6.047460 | -4.96555 | 19.61428 | 0.2342 |
| 2 | 3 | 3.94087 | 6.761266 | -9.79967 | 17.68142 | 0.5638 |
| 3 | 4 | 3.38349 | 5.480976 | -7.75519 | 14.52218 | 0.5411 |

This result of the statistical evaluation indicates that the blood serum vimentin concentration of each of group 2, group 3, and group 4 had a significant difference (p<0.05) from the blood serum vimentin concentration of group 0 (healthy individuals).

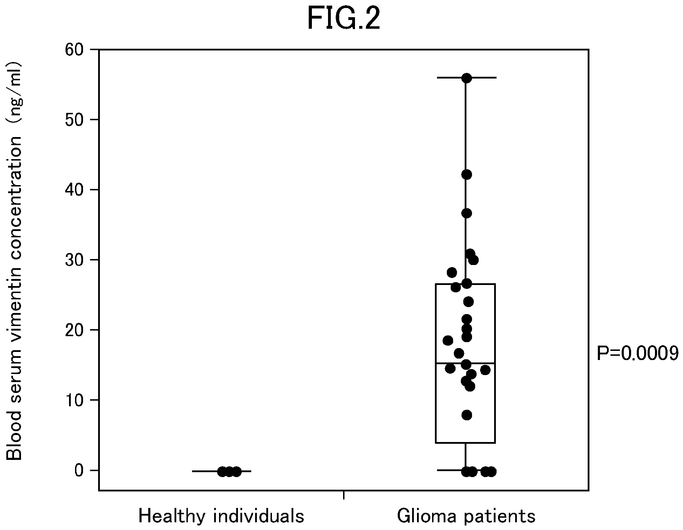

Fig. 2 shows groups 2 to 4 plotted collectively as the glioma patients.

The blood serum vimentin concentration between the healthy individuals and the glioma patients was statistically evaluated using the Student-t test. The result is shown in the following table.

**Comparison for each pair in the Student-t test**

| Level | -Level | Difference | Standard error of difference | Lower confidence limit | Upper confidence limit | p-value |
|---|---|---|---|---|---|---|
| 2 to 4 | 0 | 17.1509 | 4.7456 | 7.5263 | 26.7755 | 0.0009 |

This result of the statistical evaluation indicates that the blood serum vimentin concentrations of the glioma patients of groups 2 to 4 had a significant difference (p<0.05) from the blood serum vimentin concentrations of group 0 (healthy individuals).

From the above-described results, it is understood that glioma and healthy individuals can be distinguished using the presence of vimentin in the blood serum samples as an index.

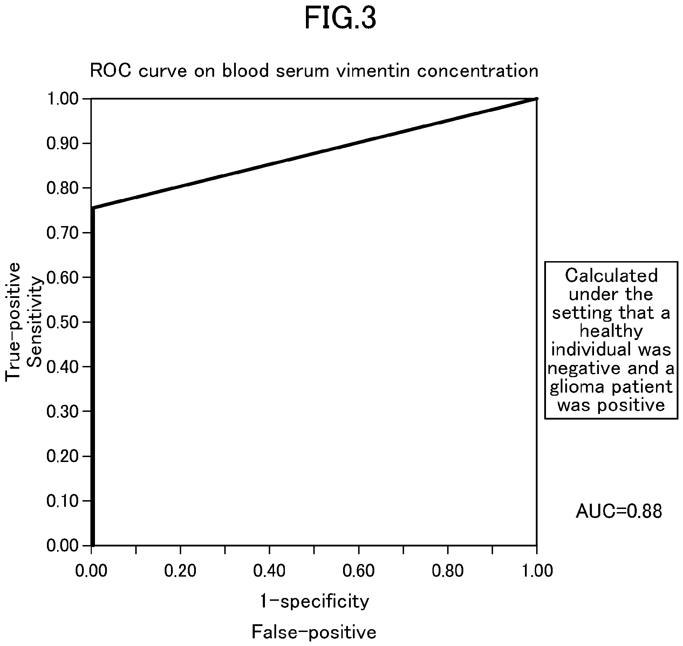

Next, a ROC curve was created using data of the blood serum vimentin concentrations of the healthy individuals (group 0) and the glioma patients (groups 2 to 4) (Fig. 3). The ROC curve indicates the reliability of the test. In a ROC curve, the "sensitivity (true-positive rate)" in the Y axis indicates the rate of indicating positive (abnormal value) when the test is conducted on a group who contract glioma, and the "1-specificity (false-positive rate)" in the X axis indicates the rate of detecting the healthy individuals (negative) who do no contract glioma as positive. Hence, the closer to 1 the area under the curve (AUC) of the ROC curve of a certain test method is, the higher the reliability of the test method is. The AUC of the ROC curve of Example 1 was 0.88. From this result, it is understood that the present invention, which uses the presence of blood vimentin as an index, is a method with a high reliability in predicting the onset of glioma.

### Industrial Applicability

The present invention, which is capable of predicting the onset of glioma, may be utilized for diagnosis of glioma.

## Claims

1. A method for predicting the onset of glioma in a subject, comprising the steps of:
(1) detecting vimentin in a blood sample derived from the subject; and
(2) in a case where vimentin is detected, predicting that there is a high possibility that the subject has contracted glioma.

2. The method according to claim 1, wherein
the glioma is glioma of grade 2 to 4.

3. The method according to claim 2, wherein
the glioma is glioma of grade 4.

4. The method according to claim 3, wherein
the glioma is glioblastoma.

5. The method according to any one of claims 1 to 4, wherein
the subject is a subject who is suspected of having glioma.

6. The method according to any one of claims 1 to 4, wherein
the subject is a subject who is not suspected of having glioma.

7. The method according to any one of claims 1 to 6, wherein
the blood sample is a blood serum sample.

8. The method according to any one of claims 1 to 7, wherein
vimentin is detected by ELISA.

9. The method according to claim 8, wherein
the ELISA is sandwich ELISA.

10. A blood marker for detecting glioma, comprising vimentin.

11. The blood marker according to claim 10, wherein
the glioma is glioma of grade 2 to 4.

12. The blood marker according to claim 11, wherein
the glioma is glioma of grade 4.

13. The blood marker according to claim 12, wherein
the glioma is glioblastoma.

14. A kit for predicting the onset of glioma, comprising:
a reagent for detecting vimentin in a blood sample; and
an instruction stating that in a case where vimentin is detected, it is predicted that there is a high possibility that the subject has contracted glioma.

15. The kit according to claim 14, wherein
the reagent for detecting vimentin contains a vimentin antibody.

16. The kit according to claim 15, wherein
the reagent for detecting vimentin further contains a reagent used in ELISA.
